# EUROPÄISCHE PATENTSCHRIFT

(11) **EP 1 732 505 B1**
(45) Veröffentlichungstag und Bekanntmachung des Hinweises auf die Patenterteilung: **24.12.2008**
(21) Anmeldenummer: 05700728.8
(22) Anmeldetag: 07.01.2005
(51) Int. Cl.: A61K 8/04, A61K 8/81, A61Q 5/12, A61Q 11/00

(54) **MITTEL ZUR OXIDATIVEN BEHANDLUNG, INSBESONDERE IN TRANSPARENTER GELFORM**
OXIDATIVE TREATMENT AGENT, PARTICULARLY IN THE FORM OF A TRANSPARENT GEL
AGENT DE TRAITEMENT OXYDATIF SE PRESENTANT NOTAMMENT SOUS FORME DE GEL TRANSPARENT

(30) Priorität: 16.01.2004 DE 102004002349
(43) Veröffentlichungstag der Anmeldung: 20.12.2006
(73) Patentinhaber: Wella Aktiengesellschaft, 64274 Darmstadt (DE)
(72) Erfinder: CASSIER, Thorsten, 64807 Dieburg (DE); LEDE, Michael, 63329 Egelsbach (DE)
(86) Internationale Anmeldenummer: PCT/EP2005/000068
(87) Internationale Veröffentlichungsnummer: WO 2005/067874

(56) Entgegenhaltungen:
- EP-A- 0 360 986
- DE-A1- 3 732 147
- DE-A1- 10 143 293
- DE-C1- 19 815 972
- FR-A- 2 818 540
- FR-A- 2 820 034
- VOL 2: "International Cosmetic Ingredient Dictionary and Handbook" 2000, THE COSMETIC, TOILETRY AND FRAGRANCE ASSOCIATION , WASHINGTON DC, US * Seite 1145 *

## Beschreibung

Die vorliegende Erfindung betrifft ein klares bzw. transparentes und gelförmiges, Mittel zur Durchführung von oxidativen Behandlungen in verschiedenen Anwendungsfeldern wie z. B. Kosmetik, Dentaltechnik, Pharmazie und Reinigungsmittel. Besonders gut geeignet ist das erfindungsgemäße Mittel für den Einsatz bei haarkosmetischen Behandlungen wie z. B. der Blondierung, der oxidativen Haarfärbung und der Fixierung bei der dauerhaften Haarverformung. Das erfindungsgemäße Mittel enthält eine Mischung aus einem Oxidationsmittel, mindestens einem Stabilisator, mindestens einem bestimmten Polymerverdicker und Wasser oder einem wässrigen Lösungsmittel.

Am Beispiel des Anwendungsfeldes Haarkosmetik soll im folgenden der Bedarf an einem solchen Mittel dargestellt werden. In der Haarkosmetik werden für verschiedene Anwendungen oxidationsmittelhaltige Haarbehandlungsmittel eingesetzt, wobei das gebräuchlichste darin enthaltene Oxidationsmittel Wasserstoffperoxid ist. Diese oxidativen Haarbehandlungsmittel spielen bei einer Reihe von haarkosmetischen Behandlungen eine wichtige Rolle. Beispielsweise werden bei der Blondierung die Haarpigmente durch Einwirkung von oxidationsmittelhaltigen Formulierungen oxidativ abgebaut und so eine Aufhellung des Haares herbeigeführt.

Die Blondierungsmittel bestehen in der Regel aus zwei Komponenten, einer Wasserstoffperoxidformulierung und einer basischen Komponente. Je nach gewünschter Aufhellleistung können zur Verstärkung Peroxodisulfate eingesetzt werden. Da wässrige Wasserstoffperoxidlösungen nur sehr schwer gezielt auf das Haar aufzubringen sind, werden mehr oder weniger stark verdickte oxidierende Emulsionen verwendet. Konsistenzgeber dieser Emulsionen sind meist längerkettige Fettalkohole, wobei an Emulgatoren meist Fettalkoholethoxylate, Alkylsulfate, Alkylethersulfate u. a. eingesetzt werden. Diese weißen Förmulierungen können zwar gut auf dem Haar lokalisiert werden, es ist dem Friseur jedoch nur sehr eingeschränkt möglich, nach der Auftragung den Haarzustand durch die sich auf dem Haar befindliche Formulierung hindurch zu sehen. Um jedoch während der Einwirkung der aufhellenden Formulierung auf das Haar eine Kontrolle über die Aufhellleistung zu haben, also durch die aufgetragene Formulierung hindurch den Aufhellungsgrad der Haare jederzeit beobachten zu können, wäre es wünschenswert, dass die Formulierung transparent und damit durchsichtig ist. Gleichzeitig sollte die Formulierung je nach Produktausführung mehr oder weniger stark verdickt sein, damit sie gezielt am Haar appliziert werden kann, nicht abläuft und daher nur in geringerem Maße auf die Haut gelangt, wodurch die Akzeptanz verbessert und das Risiko von Hautreizungen vermindert wird.

Diese Eigenschaften (Transparenz und angedickte Konsistenz) wären auch bei weiteren Haarbehandlungen wünschenswert wie z. B. bei der permanenten Haarverformung unter Verwendung von reduktionsmittelhaltigen Formulierungen. Bei der dauerhaften Haarverformung werden die Haare in der Regel zunächst mit einem Verformungsmittel auf der Basis einer keratinreduzierenden Mercaptoverbindung, welche eine Öffnung der Disulfidbrücken des Haarkeratins bewirkt, behandelt und sodann in die gewünschte Form gebracht. Als Verformungsmittel werden in der Regel keratinreduzierende Mercaptoverbindungen, wie zum Beispiel Salze oder Ester von Mercaptocarbonsäuren, verwendet. Anschließend wird das Haar mit Wasser gespült und sodann mit einem Fixiermittel oxidativ nachbehandelt. Hierbei werden die zuvor gespaltenen Disulfidbrücken in der neuen Form wieder verknüpft.

Bei bestimmten Ausführungsformen der permanenten Haarverformung wird das Haar zunächst mit einer reduzierenden Creme, in der Regel einer thioglykolathaltigen Creme, behandelt und dann mit geeigneten Hilfsmitteln wie Klammern oder auch bestimmten Wickelkörpern in die gewünschte Form gebracht. Nach einer, je nach Haarzustand variierenden Einwirkzeit wird das Haarverformungsmittel auf Thioglykolatbasis ausgespült und anschließend eine oxidationsmittelhaltige Formulierung zur Fixierung der Haare aufgebracht, wobei das Oxidationsmittel in der Regel Wasserstoffperoxid ist. Konsistenzgeber dieser Emulsionen sind meist längerkettige Fettalkohole, wobei an Emulgatoren meist Fettalkoholethoxylate, Alkylsulfate und Alkylethersulfate eingesetzt werden.

Der Nachteil dieser Emulsionen ist auch hier, dass sie nicht klar und durchsichtig, sondern weiß und trüb sind. Es ist daher dem Friseur nur sehr eingeschränkt möglich, nach der Auftragung der Formulierung Formveränderungen des Haares zu sehen. Damit man jedoch während der Einwirkung der fixierenden Formulierung auf das Haar eine optimale Kontrolle über die Formgebung hat, also durch die aufgetragene Formulierung hindurch die Form der Haare jederzeit beobachten kann, wäre es wünschenswert, dass die Formulierung transparent ist. Gleichzeitig sollte auch hier die Formulierung, je nach Produktausführung, mehr oder weniger stark verdickt sein, sowie salzstabil sein, damit sie gezielt am Haar appliziert werden kann und nicht abläuft. Besonders günstig ist es, wenn das Mittel zur oxidativen Behandlung des Haares überhaupt nicht vom Wickel abläuft, so dass die Fixierung am Friseurkunden aufrecht sitzend und während der Einwirkungszeit ohne die übliche Verwendung eines Waschbeckens durchgeführt werden kann.

Langzeitverdickungen von oxidationsmittelhaltigen Formulierungen mit polymeren Verdickern, die transparente Gele bilden, haben eine destabilisierenden Einfluss auf die zum Einsatz gelangenden Oxidationsmittel. Dies führt entweder anwendungstechnisch zwangsläufig zu schlechtere Ergebnisse infolge eines reduzierten Oxidationsmittelgehaltes oder aber zu gefährlichen Deformationen bis hin zur Zerstörung des Behälters (sogenannten Bombagen) dieser Mittel. Setzt man das am häufigsten verwendete Oxidationsmittel Wasserstoffperoxid ein, musste bisher zur Stabilisierung des Wasserstoffperoxids ein pH-Wert von 2 bis 3 eingestellt werden. Daraus ergibt sich ein weiteres Problem: Bei diesen geringen pH-Werten gibt es bisher keine geeigneten verdikkenden Polymere, die dem Mittel eine genügende Viskosität geben, gleichzeitig eine transparente Formulierung liefern sowie über 6 Monate bei 40°C viskositätsstabil, peroxidstabil und pH-stabil sind.

Die FR 2 818 540 A1 offenbart eine kosmetische und/oder dermatologische Zusammensetzung, die für die Behandlung von Keratinsubstanzen vorgesehen ist, welche in einem für Keratinsubstanzen geeigneten Träger enthält: (a) mindestens ein amphiphiles Polymer, das mindestens ein Monomer mit ethylenisch ungesättigter Bindung, das eine Sulfonsäuregruppe in freier Form oder ganz oder teilweise neutralisiert, aufweist, und (b) mindestens ein Oxidationsmittel. Die dort gestellte Aufgabe ist, transparente Gele auf der Basis von Oxidationsmitteln, insbesondere Wasserstoffperoxid, zur Verfügung zu stellen, welche nicht ablaufen, lagestabil sind und es ermöglichen, Haare kräftig und gleichmäßig zu entfärben. Die Beispiele 1 und 2 offenbaren oxidierende Zusammensetzungen, welche Wasserstoffperoxid, Tetranatrium-pyrophosphat, Natriumstannat und Acrylamido-2-methyl-2-propansulfonsäure/n-Dodecylacrylamid-Copolymer (3,5%/96,5%) zu 100% mit Natronlauge neutralisiert, enthalten.

Es bestand daher die Aufgabe, ein Mittel zur Durchführung der oxidativen Behandlung insbesondere von Haaren zur Verfügung zu stellen, das die vorstehenden Nachteile nicht aufweist, d. h. eine gelförmige Viskosität besitzt, gleichzeitig transparent ist, nicht vom Haar abtropft und über einen Zeitraum von 6 Monaten bei 40°C eine gleichbleibende Viskosität, einen gleichbleibenden pH-Wert und einen konstanten Peroxidgehalt hat.

Diese Aufgabe kann nicht gelöst werden, indem man eine dem Fachmann naheliegende Vorgehensweise wählt und einfach eine wässrige Wasserstoffperoxidlösung mit einem geeignet erscheinenden Verdicker andickt. Solche Mischungen weisen nicht die erforderliche Langzeitstabilität über 6 Monate bei 40°C auf.

Überraschenderweise wurde nunmehr gefunden, dass die gestellte Aufgabe in hervorragender Weise gelöst wird, wenn das transparente, gelförmige Mittel zur oxidativen Behandlung mindestens ein Oxidationsmittel, mindestens einen bestimmten Peroxidstabilisator, mindestens einen bestimmten Polymerverdicker und Wasser oder ein wässriges Lösungsmittel enthält.

Gegenstand der vorliegenden Erfindung ist daher ein Mittel nach Anspruch 1.

Als Oxidationsmittel kann jede beliebige, bisher in Mitteln zur oxidativen Behandlung z. B. von Haaren verwendete oxidierende Verbindung eingesetzt werden. Beispiel für derartige oxidierende Verbindungen sind Wasserstoffperoxid, Alkalibromate, wie zum Beispiel Ammoniumbromat, Erdalkalibromate, Alkalipersulfate, Erdalkalipersulfate, Ammoniumpersulfat, Alkaliperborate, Erdalkaliperborate, Ammoniumperborat, Alkalipercarbonate, Erdalkalipercarbonate, Ammoniumperborat, Calciumperoxid und Natriumjodat. Vorzugsweise wird Wasserstoffperoxid in unterschiedlichen Qualitäten (z. B. kosmetische Standardqualität, besonders eine hoch stabilisierte Qualität) eingesetzt. Die in dem Fixiermittel enthaltene Menge des Oxidationsmittels ist in Abhängigkeit von der Anwendungsdauer und Anwendungstemperatur unterschiedlich. Üblicherweise ist das Oxidationsmittel in einer Menge von 0,1 bis 25 Gew.%, vorzugsweise in einer Menge von 2 bis 14 Gew.% und besonders bevorzugt in einer Menge von 4 bis 12 Gew.% enthalten.

Der pH-Wert der Formulierung weist, insbesondere bei Verwendung von Peroxid, einen pH Wert von 2 bis 6 auf, vorzugsweise von pH 3 bis 5. Der pH-Wert bei Verwendung von Bromaten beträgt vorzugsweise von 6 bis 9, besonders bevorzugt von 7 bis 8,5.

Das erfindungsgemäße Mittel enthält weiterhin einen oder mehrere Stabilisatoren, vorzugsweise ausgewählt aus Dialkalihydrogenphosphaten, p-Acetamidophenol, Oxyquinolinsalzen, Salizylsäure und deren Salzen, 1-Hydroxyethan-1,1-diphosphonsäure, Tetranatrium-1-hydroxyethan-1,1-diphosphonat (CAS 3794-83-0; CTFA: TETRASODIUM

ETIDRONATE), Tetranatrium-imino-disuccinat (CAS 144538-83-0; CTFA: TETRASODIUM IMINODISUCClNATE), Ethylendiamin-tetranatriumacetat (INCI: EDTA) und N-(4 Ethoxyphenyl)-acetamid (CTFA: PHENACETIN).

Besonders geeignete Peroxidstabilisatoren sind solche der nachfolgenden Tabelle.

| Nr. | CTFA / INCI-Name | chemische Bezeichnung |
|---|---|---|
| (1) | DISODIUM PHOSPATE | Dinatriumhydrogenphosphat |
| (2) | ACETAMINOPHEN | p-Acetamidophenol |
| (3) | OXYQUINOLINE SULFATE | 8-Hydroxychinolinsulfat |
| (4) | SALICYLIC ACID | Salizylsäure und deren Salze |
| (5) | ETIDRONIC ACID | 1-Hydroxyethan-1,1-diphosphonsäure und deren Tetranatriumsalz |
| (6) | TETRASODIUM IMINODISUCCINATE | Tetranatrium-iminodisuccinat |
| (7) | PHENACETIN | N-(4-Ethoxyphenyl)-acetamid |

Die Peroxidstabilisatoren sind, alleine oder im Gemisch, vorzugsweise in einer Konzentration von 0,01 bis 2 Gew.% enthalten, besonders bevorzugt in einer Menge von 0,05 bis 0,3 Gew.%.

Bevorzugt sind die Kombinationen aus den Peroxidstabilisatoren
ETIDRONIC ACID und SALICYLIC ACID,
ETIDRONIC ACID und DISODIUM PHOSPATE,
TETRASODIUM ETIDRONATE und SALICYLIC ACID,
TETRASODIUM ETIDRONATE und SALICYLIC ACID,
TETRASODIUM ETIDRONATE und DISODIUM PHOSPATE
enthalten.

Besonders bevorzugt sind die in Kombination enthaltenen Peroxidstabilisatoren in den nachfolgenden Mengen enthalten:
0,1 Gew.% ETIDRONIC ACID und 0,1 Gew.% SALICYLIC ACID,
0,05 Gew.% ETIDRONIC ACID und 0,15 Gew.% Disodium Phosphat,
0,05 Gew.% TETRASODIUM ETIDRONATE und 0,15 Gew.% SALICYLIC ACID,
0,15 Gew.% TETRASODIUM ETIDRONATE und 0,05 Gew.% SALICYLIC ACID,
0,1 Gew.% TETRASODIUM ETIDRONATE und 0,1 Gew.% DISODIUM PHOSPATE.

Wenn nur ein Peroxidstabilisator enthalten ist, so ist dieser bevorzugt ausgewählt aus TETRASODIUM ETIDRONATE, Salicylsäure oder ETIDRONIC ACID, wobei die Einsatzkonzentration bevorzugt jeweils 0,2 Gew.% beträgt.

Die bevorzugten Polymerverdicker sind:

| Nr. | INCI / CTFA-Name | chemische Bezeichnung |
|---|---|---|
| (9) | ACRYLATES/C10-30 ALKYL LATE CROSSPOLYMER | Copolymer aus C₁₀₋₃₀-Alkyl-acrylat und Acrylsäure, Methacrylsäure oder deren einfachen Estern, vernetzt mit einem Allylether der Sucrose oder einem Allylether des Pentaerythriols |
| (10) | ACRYLATES/ACRYLAMIDE COPOLYMER | Copolymer aus Acrylamid und Acrylsäure, Methacrylsäure oder deren einfachen Estern (CAS No. 9003-06-9) |
| (11) | AMMONIUM ACRYLOYLDIMETHYLTAURATEBEHENETH-25 METHACRYLATE COPOLYMER | Copolymer aus Ammoniumacryloyldimethyltaurat und Beheneth-25-methacrylat |
| (12) | AMMONIUM ACRYLOYLDIMETHYL TAURATE/VP COPOLYMER | Copolymer aus Ammoniumacryloyldimethyltaurat und Vinylpyrrolidon |
| (13) | CARBOMER | Homopolymer aus Acrylsäure, Methacrylsäure, vernetzt mit einem Allylether der Sucrose, des Pentaerythriols oder des Propylens |
| (14) | HYDROXYPROPYL STARCH PHOSPHATE | Hydroxypropylether von Distärkephosphat |
| (15) | POLYQUATERNIUM-44 | Copolymer von 3-Methyl-1-vinyl-1 H-imidazolium-methyl-sulfat und 1-Vinyl-2-pyrrolidon |
| (16) | POLYQUATERNIUM-37 | N,N,N-Trimethyl-2-[(methyl-1-oxo-2-propenyl)oxy]-chlorid, Homopolymer |
| (17) | POLYQUATERNIUM-37, MINERAL OIL, SORBITANE OLEATE, PEG-1/PPG-6 TRIDECCETH 6, C10-12 ALKANE/CYCLOALKANE | Gemisch aus N,N,N-Trimethyl-2-[(methyl-1-oxo-2-propenyl)oxy]-chlorid. Homopolymer, Sorbitanmonooleat, Polyethylenglykol/Polypropylenglykoltridecylether, C₁₀₋₁₂ -Alkanen und Cycloalkanen (CAS 64742-48-9) |
| (18) | POLYQUATERNIUM-37, SORBITAN OLEATE, PROPYLENE GLYCOL DICAPRYLATE/DICAPRATE, PPG-1 TRIDECETH-6, C10-12 ALKANE/-CYCLOALKANE | Gemisch aus N,N,N-Trimethyl-2-[(methyl-1-oxo-2-propenyl)oxy]-chlorid, Homopolymer; Sorbitan-monooleat, Propylenglykol-Dicaprylsäureester (EINECS 271-516-3), Polypropylenglykoltridecylether, C₁₀₋₁₂ -Alkanen und Cycloalkanen (CAS 64742-48-9) |
| (19) | SODIUM MAGNESIUM SILICATE | Natriummagnesiumsilikat |

Die Polymerverdicker sind alleine oder im Gemisch bevorzugt in einer Menge von 1,5 bis 2,5 Gewichtsprozent enthalten.

Das erfindungsgemäße Mittel enthält weiterhin Wasser oder eine wässrige Lösung. Das Wasser ist bevorzugt in einer Menge von 50 bis 98 Gew.%, besonders bevorzugt in einer Menge von 65 bis 85 Gew.% und ganz besonders bevorzugt in einer Menge von 65 bis 80 Gew.% enthalten.

In dem erfindungsgemäßen Mittel, z. B. zur oxidativen Behandlung von Haaren, können kationische Polymere enthalten sein, vorzugsweise in einer Menge von 0,1 bis 2 Gewichtsprozent, wobei beispielsweise die folgenden kationischen Polymere oder Mischungen dieser kationischen Polymere geeignet sind: kationische Cellulosederivate, wie zum Beispiel kationische Celluloseether (beispielsweise CTFA: POLYQUATERNIUM-10), Polydimethylaminoethylmethacrylat (zu 75% mit Dimethylsulfat oder zu 100% mit Methylchlorid oder Methylbromid quatemisiert), beta-Methacryloxyethyltrimethylammoniummethosulfat-Homopolymere (CTFA: POLYQUATERNIUM-14), beta-Methacryloxyethyltrimethylammoniummethosulfat/Acrylamid-Copolymere (CTFA: POLYOUATERNIUM-5), beta-Methacryloxyethyl-trimethylammoniummethosulfat/Vinylpyrrolidon-Copolymere (CTFA: POLYQUATERNIUM-11), N-Vinylpyrrolidon/- Methacrylamidopropyltrimethylammoniumchlorid-Copolymere und kationische Chitosanderivate.

Unter diesen kationischen Polymeren sind die folgenden Verbindungen besonders bevorzugt: Polydimethylaminoethylmethacrylat (zu 75% mit Dimethylsulfat quatemisiert), N-Vinylpyrrolidon/Methacrylamidopropyltrimethylammoniumchlorid-Copolymere, beta-Methacryloxyethyl-trimethylammoniumethosulfat/Vinylpyrrolidon-Copolymere und beta-Methacryloxyethyltrimethylammoniummethosulfat-Homopolymere.

In dem erfindungsgemäßen Mittel, insbesondere zur oxidativen Behandlung von Haaren, können zusätzlich nichtionogene oberflächenaktive Verbindungen enthalten sein, wie z. B. Fettalkoholpolyglykolether mit niedrigem Ethoxylierungsgrad, beispielsweise mit 1 bis 5 Ethylenoxideinheiten im Molekül oxethylierte Alkylphenole oder oxethylierte Sorbitanfettsäureester. Die nichtionogenen oberflächenaktiven Verbindungen sind in dem Mittel vorzugsweise in einer Gesamtmenge von 0,1 bis 6 Gewichtsprozent enthalten.

Im Mittel können auch amphotere oberflächenaktive Verbindungen enthalten sein, vorzugsweise in einer Konzentration von 0,1 bis 3 Gewichtsprozent, wobei beispielsweise die folgenden amphoteren oberflächenaktiven Verbindungen oder Mischungen dieser amphoteren oberflächenaktiven Verbindungen geeignet sind: Carboxylderivate des Imidazols, beispielsweise, CTFA: COCOAMPHODIPROPIONATE, CTFA: SODIUM COCOAMPHOACETATE oder CTFA: COCOBETAINAMIDO AMPHOPROPIONA-TE (CAS 100085-64-1); N-Alkylbetaine und N-Alkylamidobetaine, N-Alkylsulfobetaine, N-Alkylaminopropionate, Alkyldimethylcarboxymethylammoniumsalze mit 12 bis 18 Kohlenstoffatomen sowie Fettsäurealkylamidobetaine, beispielsweise Fettsäureamidopropyldimethylaminoessigsäurebetain.

Selbstverständlich kann das erfindungsgemäße Mittel für derartige Mittel übliche Zusatzstoffe wie Farbstoffe oder Alkohole, beispielsweise Ethanol, Propanol, Isopropanol, Ethylenglykol, 1,2-Propylenglykol und 1,2,3-Propantriol (Glycerin); Lösungsvermittler, Puffersubstanzen, Parfümöle, Entschäumer sowie haarkonditionierende oder haarpflegende Bestandteile, wie zum Beispiel Lanolinderivate, Cholesterin oder Betain, enthalten. Weiterhin können dem erfindungsgemäßen Mittel weitere Zusatzstoffe, beispielsweise Quell- und Penetrationsstoffe, wie zum Beispiel Harnstoff, 2-Pyrrolidon, 1-Methyl-2-pyrrolidon und Dipropylenglykolmonomethylether zugesetzt werden. Die Zusatzstoffe sind in einer Menge von 0,1 bis 50 Gew.%, vorzugsweise 1 bis 20 Gew.% enthalten. Die Einsatzmenge für die Alkohole beträgt vorzugsweise 5 bis 15 Gew.%.

Das erfindungsgemäße Mittel besitzt vorzugsweise eine Viskosität von 100 bis 30.000 mPa·s, besonders bevorzugt eine Viskosität von 300 bis 18.000 mPa·s und ganz besonders bevorzugt eine Viskosität von 300 bis 5.000 mPa·s, jeweils gemessen bei 25° Celsius. Die Viskositätsangaben beziehen sich auf die Messung mit einem Haake Rotations-Viskosimeter Typ VT 550 bei einer Schergeschwindigkeit von 12,9 pro Sekunde. Es wurde eine Doppelspalt-Zylindermesseinrichtung Typ NV bzw. MV verwendet.

Das erfindungsgemäße Mittel zur Durchführung einer oxidativen Behandlung kann auch in Form eines Zweikomponentenpräparates vorliegen und wird dann erst unmittelbar vor der Anwendung durch Vermischen des reinen Polymerverdickers oder einer homogenen, den Polymerverdicker enthaltenden Zusammensetzung (Komponente 1) mit einer wässrigen Lösung des Oxidationsmittels, vorzugsweise einer Wasserstoffperoxidlösung (Komponente 2), hergestellt.

Zum Entfärben oder Blondieren von Haaren werden üblicherweise ebenfalls oxidierende Zubereitungen verwendet, welche durch Auflösen einer sogenannten Blondiermittelmischung (Pulvergemisch aus Alkalisalzen und anorganischen Persalzen, wie zum Beispiel Natrium- oder Ammoniumpersulfat) in einer wässrigen Wasserstoffperoxidlösung erhalten werden.

Es bestand dabei die Aufgabe, ein lagerstabiles Mittel zum Entfärben oder Blondieren von Humanhaaren zur Verfügung zu stellen, welches vor dem Gebrauch durch einfaches Schütteln oder Anrühren mit einem verdickten, Wasserstoffperoxid enthaltenden Mittel vermischt wird und neben seiner absolut staubfreien Darreichungs- und Anwendungsform stärkste Blondierleistung bei gleichzeitig hervorragender Lagerstabilität gewährleistet.

Vor der Anwendung wird eine an sich bekannte Blondiermittelmischung mit einem wässrigen Wasserstoffperoxidgel zu einem auftragefähigen Blondiermittel vermischt, wobei dieses Vermischen in einer Schale oder durch Anschütteln in einer Auftrageflasche erfolgen kann. Das Mischungsverhältnis von Blondiermittelmischung zu Oxidationsmittel beträgt bei Verwendung eines erfindungsgemäßen 6- bis 12-prozentigen Wasserstoffperoxid-Gels 1 : 1 bis 1 : 3.

Das so erhaltene gebrauchsfertige Mittel zum Entfärben oder Blondieren von Haaren wird auf das Haar gleichmäßig aufgetragen und nach einer Einwirkungszeit von 15 bis 60 Minuten bei Raumtemperatur (20 - 25°C) beziehungsweise von 10 bis 50 Minuten bei Wärmeeinwirkung (30 - 50°C) mit Wasser ausgespült.

In einer besonderen Ausführungsform der vorliegenden Erfindung kann das erfindungsgemäße Mittel zur oxidativen Behandlung auch als oxidierende Komponente eines Oxidationshaarfärbemittels formuliert sein.

Das gebrauchsfertige Oxidationshaarfärbemittel wird durch Vermischen einer Farbträgermasse mit dem erfindungsgemäßen Mittel zur oxidativen Behandlung unmittelbar vor der Anwendung hergestellt.

Als Oxidationsmittel kommen auch in diesem Fall hauptsächlich Wasserstoffperoxid oder dessen Additionsverbindungen an Harnstoff, Melamin oder Natriumbromat in einer 1 bis 12-prozentigen, vorzugsweise 6-prozentigen, Konzentration in Betracht, wobei Wasserstoffperoxid besonders bevorzugt wird.

Die Farbträgermasse und das erfindungsgemäße Mittel zur oxidativen Behandlung werden hierbei im Gewichtsverhältnis von 5:1 bis 1:3 miteinander vermischt, wobei ein Gewichtsverhältnis von 1:1 bis 1:2 besonders bevorzugt ist.

Für die Einstellung des jeweiligen pH-Wertes der Farbträgermasse und des erfindungsgemäßen Mittels zur oxidativen Behandlung können, je nach dem gewünschten pH-Wert, verdünnte organische oder anorganische Säuren, wie zum Beispiel Phosphorsäure, Ascorbinsäure und Milchsäure, oder Alkalien wie zum Beispiel Monoethanolamin, Triethanolamin, 2-Amino-2-methyl-1-propanol, Ammoniak, Natronlauge, Kalilauge und Tris(hydroxymethyl)amino-methan, verwendet werden.

Nach der Vermischung der Farbträgermasse mit dem Mittel zur oxidativen Behandlung wird eine für die Haarfärbebehandlung ausreichende Menge, je nach Haarfülle im allgemeinen etwa 60 bis 200 g, des erhaltenen gebrauchsfertigen Oxidationshaarfärbemittels auf das Haar aufgetragen.

Bevorzugt kann das Mittel zur oxidativen Behandlung ein Mittel zur Fixierung von Haaren nach einer reduktiven Dauerverformungsbehandlung (Dauerwellung oder Haarglättung) sein.

Die Anwendung des Fixiermittels erfolgt nach einer für die dauerhafte Haarverformung ausreichenden Einwirkungszeit des reduktiven Verformungsmittels (z.B. einer 10-prozentigen Ammoniumthioglykolatlösung vom pH-Wert 8,5). Die Einwirkungszeit des reduktiven Verformungsmittels beträgt je nach der Haarbeschaffenheit, dem pH-Wert und der Verformungswirksamkeit des Mittels sowie in Abhängigkeit von der Anwendungstemperatur etwa 5 bis 45 Minuten (5 bis 20 Minuten mit Wärmeeinwirkung; 20 bis 45 Minuten ohne Wärmeeinwirkung). Das Haar wird zunächst mit Wasser gespült und sodann mit etwa 20 bis 300 g, vorzugsweise mit 60 bis 150g des erfindungsgemäßen Fixiermittels (vorzugsweise nach den Beispielen 15 bis 27) oxidativ nachbehandelt. Nach einer Einwirkungszeit des Fixiermittels von etwa 1 bis 20 Minuten, vorzugsweise 6 bis 10 Minuten, werden die Wickler entfernt und das abgewickelte Haar, falls erforderlich, nochmals mit dem Fixiermittel 1 bis 5 Minuten lang oxidativ nachbehandelt. Sodann wird das Haar mit Wasser gespült, zur Frisur gelegt und getrocknet.

Das so behandelte Haar besitzt eine gleichmäßige und haltbare Umformung und ist hervorragend konditioniert.

Die Vorteile des erfindungsgemäßen Mittelsbestehen in der Viskositätserhöhung, der unproblematischeren Applikation, insbesondere in der Klarheit der Formulierung, die eine visuelle Kontrolle der Wirkung auf dem Haar erlaubt, in der Langzeitstabilität der Formulierungen ohne unerwünschten Peroxidabbau, unerwünschte Viskositätsschwankungen oder unerwünschte Änderung des pH-Wertes während der Lagerung.

Die folgenden Beispiele sollen den Gegenstandes der Erfindung näher erläutern.

### BEISPIELE

### Beispiel 1

| | |
|---|---|
| AMMONIUM ACRYLOYLDIMETHYL TAURATE/VP COPOLYMER (Nr. 12) | 2,00 g |
| Wasserstoffperoxid | 10,00 g |
| EDITRONIC ACID (Nr. 5) | 2,05 g |
| Dinatriumhydrogenphosphat | 0,04 g |
| 1,2,3-Propantriol | 12,00 g |
| Phosphorsäure | 0,14 g |
| Wasser | ad 100,00 g |

Die Viskosität des Gels beträgt 9.500 mPa ·s bei 25°C und der pH-Wert ist 3,5.

### Beispiel 2

| | |
|---|---|
| AMMONIUM ACRYLOYLDIMETHYLTAURATE/BEHENETH-25 METHACRYLATE COPOLYMER (Nr. 11) | 2,00 g |
| Wasserstoffperoxid | 12,00 g |
| Salizylsäure | 0,05 g |
| Dinatriumhydrogenphosphat | 0,15 g |
| Phosphorsäure | 0,13 g |
| Wasser vollentsalzt | ad 100,00 g |

Die Viskosität des Gels beträgt 15.000 mPa·s bei 25°C und der pH-Wert ist 4,0.

### Beispiel 3

| | |
|---|---|
| ACRYLATES/C₁₀₋₃₀ ALKYL ACRYLATE CROSSPOLYMER (Nr. 12) | 2,10 g |
| Wasserstoffperoxid | 11,00 g |
| EDITIDRONIC ACID (Nr. 5) | 0,15 g |
| Dinatriumhydrogenphosphat | 0,05 g |
| 1,2,3-Propantriol | 10,00 g |
| Natriumhydroxid | 0,09 g |
| Wasser vollentsalzt | ad 100,00 g |

Die Viskosität des Gels beträgt 9.500 mPa·s bei 25°C und der pH-Wert ist 3,5.

### Beispiel 4

| | |
|---|---|
| POLYQUATERNIUM-37 (Nr. 16) | 2,50 g |
| Wasserstoffperoxid | 12,00 g |
| Salizylsäure | 0,10 g |
| ACETAMINOPHEN (Nr. 2) | 0,10 g |
| 1,2,3-Propantriol | 10,00 g |
| Phosphorsäure | 0,12 g |
| Wasser vollentsalzt | ad 100,00 g |

Die Viskosität des Gels beträgt 7.500 mPa·s bei 25°C und der pH-Wert ist 3,5.

### Beispiel 5

| | |
|---|---|
| POLYQUATERNIUM 37, MINERALOIL, SORBITANE OLEATE, PPG-1/PPG-6 TRIDECCETH 6, C₁₀₋₁₂ALKANElCYCLOALKANE (Nr. 17) | 2,50 g |
| Wasserstoffperoxid | 12,00 g |
| Salizylsäure | 0,15 g |
| OXYQUINOLINE SULFATE (Nr. 3) | 0,05 g |
| 1,2,3-Propantriol | 15,00 g |
| Phosphorsäure | 0,09 g |
| Wasser vollentsalzt | ad 100,00 g |

Die Viskosität des Gels beträgt 6.800 mPa·s bei 25°C und der pH-Wert ist 3,5.

### Beispiel 6

| | |
|---|---|
| AMMONIUM ACRYLOYLDIMETHYLTAURATE/BEHENETH-25 METHACRYLATE COPOLYMER (Nr. 11) | 2,00 g |
| Wasserstoffperoxid | 9,00 g |
| Salizylsäure | 0,10 g |
| ACETAMINOPHEN (Nr. 2) | 0,10 g |
| 1,2,3-Propantriol | 10,00 g |
| Phosphorsäure | 0,17 g |
| Wasser vollentsalzt | ad 100,00g |

Die Viskosität des Gels beträgt 23.500 mPa·s bei 25°C und der pH-Wert ist 4,0.

### Beispiel 7

| | |
|---|---|
| AMMONIUM ACRYLOYLDIMETHYLTAURATE/BEHENETH-25 METHACRYLATE COPOLYMER (Nr. 11) | 1,00 g |
| AMMONIUM ACRYLOYLDIMETHYL TAURATE/VP COPOLYMER (Nr. 12) | 1,00 g |
| Wasserstoffperoxid | 10,00 g |
| Salizylsäure | 0,05 g |
| Dinatriumhydrogenphosphat | 0,15 g |
| 1,2,3-Propanetriol | 10,00 g |
| Phosphorsäure | 0,11 g |
| Wasser vollentsalzt | ad 100,00 g |

Die Viskosität des Gels beträgt 16.000 mPa·s bei 25°C und der pH-Wert ist 3,5.

### Beispiel 8

| | |
|---|---|
| ACRYLATES/C10-30 ALKYL ACRYLATE CROSSPOLYMER (Nr. 9) | 1,00 g |
| AMMONIUM ACRYLOYLDIMETHYLTAURATE/ BEHENETH-25 METHACRYLATE COPOLYMER (Nr. 11) | 1,00 g |
| Wasserstoffperoxid | 10,00 g |
| Salizylsäure | 0,05 g |
| Dinatriumhydrogenphosphat | 0,15 g |
| mit 35 Ethylenoxidgruppen oxethyliertes Rizinusöl | 0,50.g |
| mit 40 Ethylenoxidgruppen oxethyliertes hydriertes Rizinusöl | 1,00 g |
| Parfümöl | 0,15 g |
| 1,2-Propylenglykol | 2,00 g |
| Ethanol | 2,00 g |
| Phosphorsäure | 0,11 g |
| Wasser vollentsalzt | ad 100,00 g |

Die Viskosität des Gels beträgt 19.500 mPa·s bei 25°C und der pH-Wert ist 3,5.

### Beispiel 9

| | |
|---|---|
| ACRYLATES/C10-30 ALKYL ACRYLATE CROSSPOLYMER (Nr. 9) | 2,00 g |
| Wasserstoffperoxid | 10,00 g |
| Salizylsäure | 0,05 g |
| Dinatriumhydrogenphosphat | 0,15 g |
| 1,2,3-Propantriol | 10,00 g |
| mit 35 Ethylenoxidgruppen oxethyliertes Rizinusöl | 0,50 g |
| mit 40 Ethylenoxidgruppen oxethyliertes hydriertes Rizinusöl | 1,90 g |
| Parfümöl | 0,15 g |
| 1,2-Propylenglykol | 2,00 g |
| Harnstoff | 2,00 g |
| Isopropanol | 2,00 g |
| Phosphorsäure | 0,15 g |
| Wasser vollentsalzt | ad 100,00 g |

Die Viskosität des Gels beträgt 13.000 mPa·s bei 25°C und der pH-Wert ist 4,0.

### Beispiel 10

### Komponente 1

| | |
|---|---|
| AMMONIUM ACRYLOYLDIMETHYLTAURATE/BEHENETH-25 METHACRYLATE COPOLYMER (Nr. 11) | 2,00 g |

### Komponente 2

| | |
|---|---|
| Wasserstoffperoxid | 9,00 g |
| Salizylsäure | 0,10 g |
| ACETAMINOPHEN (Nr. 2) | 0,10 g |
| 1,2,3-Propantriol | 10,00 g |
| Phosphorsäure | 0,17 g |
| Wasser vollentsalzt | ad 98,00 g |

Die Komponenten 1 und 2 werden unmittelbar vor der Anwendung miteinander vermischt. Die Viskosität des Gels beträgt 22.000 mPa·s bei 25°C und der pH-Wert ist 3,5.

### Beispiel 11

### Komponente 1

| | |
|---|---|
| AMMONIUM ACRYLOYLDIMETHYLTAURATE/BEHENETH-25 METHACRYLATE COPOLYMER (Nr.11) | 2,00 g |
| 1,3-bis(Hydroxymethyl)-5,5-dimethylimidazolidin-2,4-dion (+) (CTFA: DMDM HYDANTOIN) | 0,30 g |
| Phosphorsäure | 0,20 g |
| Wasser vollentsalzt | 47,50 g |

### Komponente 2

| | |
|---|---|
| Wasserstoffperoxid | 9,00 g |
| Salizylsäure | 0,10 g |
| ACETAMINOPHEN (Nr. 2) | 0,10 g |
| 1,2,3-Propantriol | 10,00 g |
| Phosphorsäure | 0,17 g |
| Wasser vollentsalzt | ad 50,00 g |

Die Komponenten 1 und 2 werden unmittelbar vor der Anwendung miteinander vermischt. Die Viskosität des Gels beträgt 23.000 mPa·s bei 25°C und der pH-Wert ist 3,0.

### Beispiel 12

| | |
|---|---|
| AMMONIUM ACRYLOYLDIMETHYL TAURATE/VP COPOLYMER (Nr. 12) | 2,00 g |
| Wasserstoffperoxid | 6,00 g |
| EDITRONIC ACID (Nr. 5) | 2,05 g |
| Dinatriumhydrogenphosphat | 0,04 g |
| 1,2,3-Propantriof | 12,00 g |
| Phosphorsäure | 0,14 g |
| Wasser | ad 100,00 g |

Die Viskosität des Gels beträgt 9.300 mPa·s bei 25°C und der pH-Wert ist 3,5.

### Beispiel 13 Blondiermittel

### Komponente 1 Blondiermittelmischung

| | |
|---|---|
| Kaliumpersulfat | 25,00 g |
| Ammoniumpersulfat | 18,00 g |
| Natriummetasilikat | 23,00 g |
| Natriumalginat | 2,00 g |
| Xanthan Gum | 2,00 g |
| Acrylsäurepolymer (CTFA: Carbomer) | 0,50 g |
| Isopropylpalmitat | 26,50 g |
| Bienenwachs | 2,50 g |
| Ethylendiaminotetraessigsäure | 0,50 g |
| | 100,00 g |

25 g der Blondiermittelmischung (Komponente 1) werden mit 25 g eines wasserstoffperoxidhaltigen Gels (Komponente 2) der folgenden Zusammensetzung in einer Schale mit einem Pinsel homogen verrührt:

### Komponente 2 Wasserstoffperoxidgel

| | |
|---|---|
| AMMONIUM ACRYLOYLDIMETHYLTAURATE/BEHENETH-25 METHACRYLATE COPOLYMER (Nr. 11) | 2,00 g |
| Wasserstoffperoxid | 9,00 g |
| Salizylsäure | 0,05 g |
| Dinatriumhydrogenphosphat | 0,15 g |
| 1,2,3-Propantriol | 10,00 g |
| Phosphorsäure | 0,13 g |
| Wasser vollentsalzt | ad 100,00 g |

Die Viskosität des Gels beträgt 23.000 mPa -s bei 25°C und der pH-Wert ist 3,5. Das erhaltene Blondiermittel wird auf mittelbraunes Haar gleichmäßig aufgetragen und nach einer Einwirkungszeit von 30 Minuten bei Raumtemperatur mit warmem Wasser abgespült und das Haar getrocknet. Das so behandelte Haar ist auf einen hellblonden Farbton aufgehellt worden.

### Beispiel14 Oxidationshaarfärbemittel

### Komponente 1 Farbträgermasse

| | |
|---|---|
| Ethanol | 8,00 g |
| Natriumlaurylethersulfat, 28%ige wässrige Lösung | 10,00 g |
| Ammoniak, 25%ige wässrige Lösung | 9,00 g |
| Ascorbinsäure | 0,30 g |
| Natriumsulfit | 0,40 g |
| Oxidationsfarbstoffgemisch, bestehend aus mindestens einem Kuppler und mindestens einem Entwickler | 5 mol |
| Wasser | ad 100,00 g |

Unmittelbar vor der Anwendung werden jeweils 20 g der Farbstoffträgermasse (Komponente 1) und 20 g des nachfolgend angeführten 6%igen Wasserstoffperoxid-Gels (Komponente 2) miteinander vermischt und man erhält so das gebrauchsfertige Oxidationshaarfärbemittel.

### Komponente 2 Wasserstoffperoxid-Gel

| | |
|---|---|
| AMMONIUM ACRYLOYLDIMETHYL TAURATE/VP COPOLYMER (Nr. 12) | 2,00 g |
| Wasserstoffperoxid | 6,00 g |
| EDITRONIC ACID (Nr. 5) | 2,05 g |
| Dinatriumhydrogenphosphat | 0,04 g |
| 1,2,3-Propantriol | 12,00 g |
| Phosphorsäure | 0,14 g |
| Wasser | ad 100,00 g |

Die Viskosität des Gels beträgt 10.300 mPa·s bei 25°C und der pH-Wert ist 3,5.

Das so erhaltene gebrauchsfertigen Oxidationshaarfärbemittel wird anschließend auf menschliche Haare aufgetragen. Nach einer Einwirkungszeit von 30 Minuten bei 40 °C werden die Haare mit einem Shampoo gewaschen, mit Wasser gespült und getrocknet.

### Beispiel 15 Fixiermittel

| | |
|---|---|
| AMMONIUM ACRYLOYLDIMETHYL TAURATE/VP COPOLYMER (Nr. 12) | 0,60 g |
| Wasserstoffperoxid, 50%ige wäßrige Lösung | 5,00 g |
| Polyoxyethylen(20)sorbitan-monopalmitat (CTFA: POLYSORBATE 40) | 1,00 g |
| Parfüm | 0,30 g |
| EDITRONIC ACID (Nr. 5) | 0.80 g |
| Dinatriumhydrogenphosphat | 0,05 g |
| Salicylsäure | 0,15 g |
| 9,2,3-Propantriol | 1,00 g |
| Natriumhydroxid | 0,06 g |
| Phosphorsäure | 0,04 g |
| Wasser | ad 100,00 g |

Das Fixiermittel besitzt einen pH-Wert von 3,5. Die Viskosität des Mittels beträgt 450 mPa · s bei 25° C.

Die Fixierung der Haare nach der reduktiven Haarverformung erfolgt wie auf Seite 11 1 beschrieben. Das so behandelte Haar besitzt eine haltbare und gleichmäßige Umformung und ist hervorragend konditioniert.

### Beispiel 16 Fixiermittel

| | |
|---|---|
| AMMONIUM ACRYLOYLDIMETHYL TAURATE/VP COPOLYMER (Nr. 12) | 0,80 g |
| Wasserstoffperoxid | 5,00 g |
| Polyoxyethylen(20)sorbitan-monopalmitat (CTFA: POLYSORBATE 40) | 1,00 g |
| Parfüm | 0,30g |
| EDITRONIC ACID (Nr. 5) | 0,10 g |
| Dinatriumhydrogenphosphat | 0,05 g |
| Salicylsäure | 0,05 g |
| 1,2,3-Propantriol | 1,00 g |
| Natriumhydroxid | 0,14 g |
| Phosphorsäure | 0,04 g |
| Wasser | ad 100,00 g |

Das Fixiermittel besitzt eine Viskosität von 700 mPa · s bei 25° C und einen pH-Wert 4,0.

### Beispiel 17

| | |
|---|---|
| AMMONIUM ACRYLOYLDIMETHYL TAURATE/VP COPOLYMER (Nr. 12) | 0,70 g |
| Wasserstoffperoxid | 5,00 g |
| Polyoxyethylen(20)sorbitan-monopalmitat | 1,00 g |
| (CTFA: POLYSORBATE 40) | |
| Parfüm | 0,30 g |
| EDITRONIC ACID (Nr. 5) | 0.05 g |
| Dinatriumhydrogenphosphat | 0,05 g |
| Salicylsäure | 0,15 g |
| Panthenol | 0,25 g |
| Natriumhydroxid | 0,10 g |
| Phosphorsäure | 0,04 g |
| Wasser | ad 100,00 g |

Das Fixiermittel besitzt eine Viskosität von 720 mPa · s bei 25° C und einen pH-Wert 3,0.

### Beispiel 18

| | |
|---|---|
| AMMONIUM ACRYLOYLDIMETHYL TAURATE/VP COPOLYMER (NR.12) | 0,70 g |
| Wasserstoffperoxid | 5,00 g |
| Polyoxyethylen(20)sorbitan-monopalmitat (CTFA: POLYSORBATE 40) | 1,00 g |
| Parfüm . | 0,30 g |
| EDITRONIC ACID (Nr. 5) | 0,15 g |
| Salicylsäure | 0,05 g |
| PEG/PPG-25/25 DIMETHICONE | 1,00 g |
| Natriumhydroxid | 0,10 g |
| Phosphorsäure | 0,04 g |
| Wasser | ad 100,00 g |

Das Fixiermittel besitzt eine Viskosität von 680 mPa · s bei 25° C und einen pH-Wert 3,5.

### Beispiel 19

| | |
|---|---|
| POLYQUATERNIUM 37 (Nr. 16) | 0,80 g |
| Wasserstoffperoxid | 5,00 g |
| Polyoxyethylen(20)sorbitan-monopalmitat (CTFA: POLYSORBATE 40) | 1,00 g |
| Parfüm | 0,30 g |
| EDITRONIC ACID (Nr. 5) | 0,15 g |
| Salicylsäure | 0,05 g |
| 1,2,3-Propantriol | 3,00 g |
| Natriumhydroxid | 0,14 g |
| Phosphorsäure | 0,04 g |
| Wasser | ad 100,00 g |

Das Fixiermittel besitzt eine Viskosität von 700 mPa · s bei 25° C und einen pH-Wert von 3,5.

### Beispiel 20

| | |
|---|---|
| POLYQUATERNIUM 37 (Nr. 16) | 0,80 g |
| Wasserstoffperoxid | 5,00 g |
| Polyoxyethylen(20)sorbitan-monopalmitat (CTFA: POLYSORBATE 40) | 1,00 g |
| Parfüm | 0,30 g |
| EDITRONIC ACID (Nr. 5) | 0,15 g |
| Salicylsäure | 0,05 g |
| Cetyltrimethylammoniumchlorid | 0,15 g |
| Natriumhydroxid | 0,14 g |
| Phosphorsäure | 0,04 g |
| Wasser | ad 100,00 g |

Das Fixiermittel besitzt eine Viskosität von 730 mPa · s bei 25° C und einen pH-Wert von 3,5.

### Beispiel 21

| | |
|---|---|
| AMMONIUM ACRYLOYLDIMETHYLTAURATE/BEHENETH-25 METHACRYLATE COPOLYMER (Nr.11) | 0,60 g |
| Wasserstoffperoxid | 5,00 g |
| Polyoxyethylen(20)sorbitan-monopalmitat (CTFA: POLYSORBATE 40) | 1,00 g |
| Parfüm | 0,30 g |
| EDITRONIC ACID (Nr. 5) | 0,70 g |
| Dinatriumhydrogenphosphat | 0,05 g |
| Salicylsäure | 0,15 g |
| 1,2,3-Propantriol | 1,00 g |
| Natriumhydroxid | 0,06 g |
| Phosphorsäure | 0,04 g |
| Wasser | ad 100,00 g |

Das Fixiermittel besitzt eine Viskosität von 500 mPa · s bei 25° C und einen pH-Wert von 3,0.

### Beispiel 22

| | |
|---|---|
| AMMONIUM ACRYLOYLDIMETHYLTAURATE/BEHENETH-25 METHACRYLATE COPOLYMER (Nr.11) | 0,70 g |
| Wasserstoffperoxid | 5,00 g |
| Polyoxyethylen(20)sorbitan-monopalmitat (CTFA: POLYSORBATE 40) | 1,00 g |
| Parfüm | 0,30 g |
| EDITRONIC ACID (Nr. 5) | 0,20 g |
| Dinatriumhydrogenphosphat | 0,05 g |
| Salicylsäure | 0,05 g |
| 1,2,3-Propantriol | 1,00 g |
| Natriumhydroxid | 0,14 g |
| Phosphorsäure | 0,04 g |
| Wasser | ad 100,00 g |

Das Fixiermittel besitzt eine Viskosität von 750 mPa · s bei 25° C.

### Beispiel 23

| | |
|---|---|
| AMMONIUM ACRYLOYLDIMETHYLTAURATE/BEHENETH-25 METHACRYLATE COPOLYMER (Nr.11) | 0,80 g |
| Wasserstoffperoxid | 5,00 g |
| Polyoxyethylen(20)sorbitan-monopalmitat (CTFA: POLYSORBATE 40) | 1,00 g |
| Parfüm | 0,30 g |
| EDITRONIC ACID (Nr. 5) | 0,05 g |
| Dinatriumhydrogenphosphat | 0,05 g |
| Salicylsäure | 0,15 g |
| Panthenol | 0,25 g |
| Natriumhydroxid | 0,10 g |
| Phosphorsäure | 0,04 g |
| Wasser | ad 100,00 g |

Das Fixiermittel besitzt eine Viskosität von 800 mPa · s bei 25° C und einen pH-Wert von 3,5.

### Beispiel 24

| | |
|---|---|
| ACRYLATES/C10-30 ALKYL ACRYLATE CROSSPOLYMER (Nr. 9) | 0,65 g |
| Wasserstoffperoxid | 5,00 g |
| Salizylsäure | 0,05 g |
| Diriatriumhydrogenphosphat | 0,15 g |
| Polyoxyethylen(20)sorbitan-monopalmitat (CTFA: POLYSORBATE 40) | 1,00 g |
| Parfümöl | 0,15 g |
| 1,2,3-Propantriol | 1,00 g |
| Oxethyliertes Rizinusöl mit 35 Ethylenoxidgruppen | 0,50 g |
| Oxethyliertes hydriertes Rizinusöl mit 40 Ethylenoxidgruppen | 1,00 g |
| Panthenol | 0,21 g |
| Ethanol | 2,00 g |
| Natriumhydroxid | 0,12 g |
| Phosphorsäure | 0,04 g |
| Wasser vollentsalzt | ad 100,00 g |

Das Fixiermittel besitzt eine Viskosität von 450 mPa · s bei 25° C und einen pH-Wert von 3,5.

### Beispiel 25

| | |
|---|---|
| ACRYLATES/C10-30 ALKYL ACRYLATE CROSSPOLYMER (Nr. 9) | 0,75 g |
| Wasserstoffperoxid | 5,00 g |
| Polyoxyethylen(20)sorbitan-monopalmitat (CTFA: POLYSORBATE 40) | 1,00 g |
| Parfüm | 0,30 g |
| EDITRONIC ACID (Nr. 5) | 0,85 g |
| Dinatriumhydrogenphosphat | 0,50 g |
| Salicylsäure | 0,05 g |
| Oxethyliertes hydriertes Rizinusöl mit 60 Ethylenoxidgruppen (CTFA: PEG-60 HYDROGENATED CASTOR OIL) | 1,00 g |
| Parfümöl | 0,30 g |
| 1,2-Propylenglykol | 2,00 g |
| Natriumhydroxid | 0,06 g |
| Phosphorsäure | 0,04 g |
| Wasser vollentsalzt | ad 100,00 g |

Das Fixiermittel besitzt eine Viskosität von 600 mPa · s bei 25° C und einen pH-Wert von 3,5.

### Beispiel 26

| | |
|---|---|
| AMMONIUM ACRYLOYLDIMETHYLTAURATE/BEHENETH-25 METHACRYLATE COPOLYMER (Nr. 11) | 0,30 g |
| AMMONIUM ACRYLOYLDIMETHYL TAURATENP COPOLYMER (Nr. 12) | 0,32 g |
| Wasserstoffperoxid | 5,00 g |
| EDITRONIC ACID (Nr. 5) | 0,85 g |
| Salicylsäure | 0,05 g |
| Oxethyliertes hydriertes Rizinusöl mit 60 Ethylenoxidgruppen (CTFA: PEG-60 HYDROGENATED CASTOR OIL) | 1,00 g |
| Parfümöl | 0,30 g |
| Natriumhydroxid | 0,06 g |
| Phosphorsäure | 0,07 g |
| Wasser vollentsalzt | ad 100,00 g |

Das Fixiermittel besitzt eine Viskosität von 600 mPa · s bei 25° C und einen pH-Wert von 3,5.

### Beispiel 27

| | |
|---|---|
| AMMONIUM ACRYLOYLDIMETHYLTAURATE/BEHENETH-25 METHACRYLATE COPOLYMER (Nr. 11) | 0,32 g |
| ACRYLATES/C10-30 ALKYL ACRYLATE CROSSPOLYMER (Nr. 9) | 0,32 g |
| Wasserstoffperoxid | 5,00 g |
| EDITRONIC ACID (Nr. 5) | 0,85 g |
| Salicylsäure | 0,05 g |
| Oxethyliertes hydriertes Rizinusöl mit 60 Ethylenoxidgruppen (CTFA: PEG-60 HYDROGENATED CASTOR OIL) | 1,00 g |
| Parfümöl | 0,30 g |
| Natriumhydroxid | 0,06 g |
| Phosphorsäure | 0,06 g |
| Wasser vollentsalzt | ad 100,00 g |

Das Fixiermittel besitzt eine Viskosität von 750 mPa · s bei 25° C und einen pH-Wert von 4,0.

## Patentansprüche

1. Transparentes gelförmiges Mittel zur oxidativen Behandlung, enthaltend
a) mindestens ein Oxidationsmittel,
b) mindestens einen Peroxidstabilisator,
c) 0,5 bis 3,0 Gew. % mindestens eines Polymerverdickers, ausgewählt aus den Gruppen
(i) der Copolymerisate der Acrylsäure, ausgewählt aus der Gruppe: Copolymer aus C₁₀₋₃₀-Alkykl-acrylat und Acrylsäure, Methacrylsäure oder deren einfachen Estern, vernetzt mit einem Allylether der Sucrose oder einem Allylether des Pentaerythriols; Copolymer aus Acrylamid und Acrylsäure, Methacrylsäure oder deren einfachen Estern; Copolymer aus Ammoniumacryloyldimethyltaurat und Beheneth-25-methacrylat; Copolymer aus Ammoniumacryloyldimethyltaurat und Vinylpyrrolidon; und Homopolymer aus Acrylsäure, Methacrylsäure, vernetzt mit einem Allylether der Sucrose, des Pentaerythriols oder des Propylens;
(ii) der Polymere mit Propylenoxygruppen;
(iii) der Hydroxyalkyl-stärkephosphate und
(iv) der Alkalimagnesiumsilikate
sowie
d) Wasser oder ein wässriges Lösungsmittel.

2. Mittel nach Anspruch 1, **dadurch gekennzeichnet, dass** das Oxidationsmittel ausgewählt ist aus Wasserstoffperoxid, Alkalibromaten, Erdalkalibromaten, Ammoniumbromat, Alkalipersulfaten, Erdalkalipersulfaten, Ammoniumpersulfat, Alkaliperboraten, Erdalkaliperboraten, Ammoniumperborat, Alkalipercarbonaten, Erdalkalipercarbonaten, Calciumperoxid und Natriumjodat.

3. Mittel nach einem der Ansprüche 1 oder 2, **dadurch gekennzeichnet, dass** es als Oxidationsmittel Bromat enthält und einen pH zwischen 7 und 8,5 aufweist.

4. Mittel nach einem der Ansprüche 1 bis 3, **dadurch gekennzeichnet**, es einen pH Wert von 2 bis 6 aufweist.

5. Mittel nach einem der Ansprüche 1 bis 4, **dadurch gekennzeichnet, dass** das Oxidationsmittel in einer Menge von 0,1 bis 25 Gewichtsprozent enthalten ist.

6. Mittel nach einem der Ansprüche 1 bis 5, **dadurch gekennzeichnet, dass** der Stabilisator ausgewählt ist aus Dialkalihydrogenphosphaten, p-Acetamidophenol, Oxyquinolinsalzen, Salizylsäure und deren Salzen, 1-Hydroxyethan-1,1-diphosphonsäure, Tetranatrium-1-hydroxyethan-1,1-diphosphonat, Tetranatrium-imino-disuccinat, Ethylendiamin-tetranatriumacetat und N-(4-Ethoxyphenyl)-acetamid.

7. Mittel nach einem der Ansprüche 1 bis 6, **dadurch gekennzeichnet, dass** der Stabilisator in einer Menge von 0,01 bis 2 Gewichtsprozent enthalten ist.

8. Mittel nach einem der Ansprüche 1 bis 7, **dadurch gekennzeichnet, dass** der Stabilisator eine 2er Kombination, ausgewählt aus den folgenden ist:
ETIDRONIC ACID und SALICYLIC ACID,
ETIDRONIC ACID und DISODIUM PHOSPATE,
TETRASODIUM ETIDRONATE und SALICYLIC ACID,
TETRASODIUM ETIDRONATE und SALICYLIC ACID,
TETRASODIUM ETIDRONATE und DISODIUM PHOSPATE.

9. Mittel nach einem der Ansprüche 1 bis 8, **dadurch gekennzeichnet, dass** der Stabilisator in einer Menge von 0,1 bis 0,3 Gew.% enthalten ist und ausgewählt ist aus TETRASODIUM ETIDRONATE, SALICYLIC ACID und ETIDRONIC ACID.

10. Mittel nach einem der Ansprüche 1 bis 9, **dadurch gekennzeichnet, dass** der Polymerverdicker ausgewählt ist aus der nachfolgenden Tabelle:
| Nr. | INCI / CTFA-Name | chemische Bezeichnung |
|---|---|---|
| (9) | ACRYLATES/C10-30 ALKYL ACRYLATE CROSSPOLYMER | Copolymer aus C₁₀₋₃₀-Alkyl-acrylat und Acrylsäure, Methacrylsäure oder deren einfachen Estern, vernetzt mit einem Allylether der Sucrose oder einem Allylether des Pentaerythriols |
| (10) | ACRYLATES/ACRYLAMIDE COPOLYMER | Copolymer aus Acrylamid und Acrylsäure, Methacrylsäure oder deren einfachen Estern (CAS No. 9003-06-9) |
| (11) | AMMONIUM ACRYLOYLDIMETHYLTAURATE/BEHENETH-25 METHACRYLATE COPOLYMER | Copolymer aus Ammoniumacryloyldimethyltaurat und Beheneth-25-methacrylat |
| (12) | AMMONIUM ACRYLOYLDIMETHYL TAURATE/VP COPOLYMER | Copolymer aus Ammoniumacryloyldimethyltaurat und Vinylpyrrolidon |
| (13) | CARBOMER | Homopolymer aus Acrylsäure, Methacrylsäure, vernetzt mit einem Allylether der Sucrose, des Pentaerythriols oder des Propylens |
| (14) | HYDROXYPROPYL STARCH PHOSPHATE | Hydroxypropylether von Distärkephosphat |
| (15) | POLYQUATERNIUM-44 | Copolymer von 3-Methyl-1-vinyl-1 H-imidazolium-methyl-sulfat und 1 -Vinyl-2-pyrrolidon |
| (16) | POLYQUATERNIUM-37 | N,N,N-Trimethyl-2-[(methyl-1-oxo-2-propenyl)oxy]-chlorid, Homopolymer |
| (17) | POLYQUATERNIUM-37, MINERALOIL, SORBITANE OLEATE, PEG-1/PPG-6 TRIDECCETH 6, C10-12 ALKANE/CYCLOALKANE | Gemisch aus N,N,N-Trimethyl-2-[(methyl-1-oxo-2-propenyl)oxy]-chlorid, Homopolymer; Sorbitanmonooleat, Polyethylenglykol/Polypropylenglykoltridecylether, C₁₀₋₁₂ -Alkanen und Cycloalkanen (CAS 64742-48-9) |
| (18) | POLYQUATERNIUM-37, SORBITAN OLEATE, PROPYLENE GLYCOL DICAPRYLATE/DICAPRATE, PPG-1 TRIDECETH-6, C₁₀₋₁₂ ALKANE/CYCLOALKANE | Gemisch aus N,N,N-Trimethyl-2-[(methyl-1-oxo-2-propenyl)oxy]-chlorid Homopolymer; Sorbitanmonooleat, Propylenglykol-Dicaprylsäureester (EINECS 271-516-3), Polypropylenglykoltridecylether, C₁₀₋₁₂ -Alkanen und Cycloalkanen (CAS 64742-48-9) |
| (19) | SODIUM MAGNESIUM SILICATE | Natriummagnesiumsilikat |

11. Mittel nach einem der Ansprüche 1 bis 10, **dadurch gekennzeichnet, dass** das Wasser in einer Menge von 50 bis 98 Gew. % enthalten ist.

12. Mittel nach einem der Ansprüche 1 bis 11, **dadurch gekennzeichnet, dass** es 1 bis 20 Gew.% eines Alkohols enthält.

13. Mittel nach Anspruch 12, **dadurch gekennzeichnet, dass** der Alkohol 1,2,3-Propantriol ist.

14. Mittel nach einem der Ansprüche 1 bis 13, **dadurch gekennzeichnet, dass** es mindestens ein kationisches Polymer enthält.

15. Mittel nach einem der Ansprüche 1 bis 14, **dadurch gekennzeichnet, dass** es mindestens eine amphotere oberflächenaktive Verbindung enthält, welche ausgewählt ist aus Carboxylderivaten des Imidazols, N-Alkylamidobetainen, N-Alkylsulfobetainen, N-Alkylaminopropionaten, Alkyldimethylcarboxymethylammoniumsalzen mit 12 bis 18 Kohlenstoffatomen und Fettsäurealkylamidobetainen.

16. Mittel nach einem der Ansprüche 1 bis 15, **dadurch gekennzeichnet, dass** es eine Viskosität von 100 bis 30.000 mPa · s aufweist, gemessen bei 25°Celsius mit einem Haake Rotations-Viskosimeter Typ VT 550 bei einer Schergeschwindigkeit von 12,9 pro Sekunde.

17. Mittel nach einem der Ansprüche 1 bis 16, **dadurch gekennzeichnet, dass** es in Form eines 2-Komponenten-Präparates vorliegt und unmittelbar vor der Anwendung durch Vermischen des reinen Polymerverdickers oder einer den Polymerverdicker enthaltenden Zusammensetzung (Komponente 1) mit einer wässrigen Wasserstoffperoxidlösung (Komponente 2) hergestellt wird.

## Claims

1. Transparent gel-like agent for oxidative treatment, comprising
a) at least one oxidizing agent,
b) at least one peroxide stabilizer,
c) 0.5 to 3.0% by weight of at least one polymeric thickener selected from the groups
(i) the copolymers of acrylic acid, selected from the group: copolymer of C₁₀₋₃₀-alkyl acrylate and acrylic acid, methacrylic acid or monoesters thereof, crosslinked with an allyl ether of sucrose or an allyl ether of pentaerythritol; copolymer of acrylamide and acrylic acid, methacrylic acid or monoesters thereof; copolymer of ammonium acryloyldimethyltaurate and beheneth-25 methacrylate; copolymer of ammonium acryloyldimethyltaurate and vinylpyrrolidone; and homopolymer of acrylic acid, methacrylic acid, crosslinked with an allyl ether of sucrose, of pentaerythritol or of propylene;
(ii) the polymers with propyleneoxy groups;
(iii) the hydroxyalkyl starch phosphates and
(iv) the alkali metal magnesium silicates, and
d) water or an aqueous solvent.

2. Agent according to Claim 1, **characterized in that** the oxidizing agent is selected from hydrogen peroxide, alkali metal bromates, alkaline earth metal bromates, ammonium bromate, alkali metal persulphates, alkaline earth metal persulphates, ammonium persulphate, alkali metal perborates, alkaline earth metal perborates, ammonium perborate, alkali metal percarbonates, alkaline earth metal percarbonates, calcium peroxide and sodium iodate.

3. Agent according to one of Claims 1 or 2, **characterized in that** it comprises bromate as oxidizing agent and has a pH between 7 and 8.5.

4. Agent according to one of Claims 1 to 3, **characterized in that** it has a pH of from 2 to 6.

5. Agent according to one of Claims 1 to 4, **characterized in that** the oxidizing agent is present in an amount of from 0.1 to 25 per cent by weight. .

6. Agent according to one of Claims 1 to 5, **characterized in that** the stabilizer is selected from dialkali metal hydrogenphosphates, p-acetamidophenol, oxyquinoline salts, salicylic acid and salts thereof, 1-hydroxyethane-1,1-diphosphonic acid, tetrasodium 1-hydroxyethane-1,1-diphosphonate, tetrasodium iminodisuccinate, ethylenediamine tetrasodium acetate and N-(4-ethoxyphenyl)acetamide.

7. Agent according to one of Claims 1 to 6, **characterized in that** the stabilizer is present in an amount of from 0.01 to 2 per cent by weight.

8. Agent according to one of Claims 1 to 7, **characterized in that** the stabilizer is a 2-component combination selected from the following:
ETIDRONIC ACID and SALICYLIC ACID,
ETIDRONIC ACID and DISODIUM PHOSPHATE,
TETRASODIUM ETIDRONATE and SALICYLIC ACID,
TETRASODIUM ETIDRONATE and DISODIUM PHOSPHATE.

9. Agent according to one of Claims 1 to 8, **characterized in that** the stabilizer is present in an amount of from 0.1 to 0.3% by weight and is selected from TETRASODIUM ETIDRONATE, SALICYLIC ACID and ETIDRONIC ACID.

10. Agent according to one of Claims 1 to 9, **characterized in that** the polymeric thickener is selected from the following table:
| No. | INCI/CTFA Name | Chemical name |
|---|---|---|
| (9) | ACRYLATES/C10-30 ALKYL ACRYLATE CROSSPOLYMER | Copolymer of C₁₀₋₃₀-alkyl acrylate and acrylic acid, methacrylic acid or monoesters thereof, crosslinked with an allyl ether of sucrose or an allyl ether of pentaerythritol |
| (10) | ACRYLATES/ACRYLAMIDE COPOLYMER | Copolymer of acrylamide and acrylic acid, methacrylic acid or monoesters thereof (CAS No. 9003-06-9) |
| (11) | AMMONIUM ACRYLOYLDIMETHYLTAURATE/BEHENETH-25 METHACRYLATE COPOLYMER | Copolymer of ammonium acryloyldimethyltaurate and beheneth-25 methacrylate |
| (12) | AMMONIUM ACRYLOYLDIMETHYLTAURATE/-COPOLYMER | Copolymer of ammonium acryloyldimethyltaurate and vinylpyrrolidone |
| (13) | CARBOMER | Homopolymer of acrylic acid, methacrylic acid, crosslinked with an allyl ether of sucrose, of pentaerythritol or of propylene |
| (14) | HYDROXYPROPYL STARCH PHOSPHATE | Hydroxypropyl ether of distarch phosphate |
| (15) | POLYQUATERNIUM-44 | Copolymer of 3-methyl-1-vinyl-1H-imidazolium methyl sulphate and 1-vinyl-2-pyrrolidone |
| (16) | POLYQUATERNIUM-37 | N,N,N-Trimethyl-2-[((methyl-1-oxo-2-propenyl)oxy] chloride, homopolymer |
| (17) | POLYQUATERNIUM-37, MINERAL OIL, SORBITAN OLEATE, PEG-1/PPG-6 TRIDECETH 6, C10-12 ALKANE/CYCLOALKANE | Mixture of N,N,N-trimethyl-2-[(methyl-1-oxo-2-propenyl)oxy] chloride, homopolymer; sorbitan monooleate, polyethylene glycol/polypropylene glycol tridecyl ether, C₁₀₋₁₂-alkanes and cycloalkanes (CAS 64742-48-9) |
| (18) | POLYQUATERNIUM-37, SORBITAN OLEATE, PROPYLENE GLYCOL DICAPRYLATE/ DICAPRATE, PPG-1 TRIDECETH-6, C10-12 ALKANE/-CYCLOALKANE | Mixture of N,N,N-trimethyl-2-[(methyl-1-oxo-2-propenyl)oxy] chloride homopolymer; sorbitan monooleate, propylene glycol dicaprylic acid ester (EINECS 271-516-3), polypropylene glycol tridecyl ether, C₁₀₋₁₂-alkanes and cycloalkanes (CAS 64742-58-9) |
| (19) | SODIUM MAGNESIUM SILICATE | Sodium magnesium silicate |

11. Agent according to one of Claims 1 to 10, **characterized in that** the water is present in an amount of from 50 to 98% by weight.

12. Agent according to one of Claims 1 to 11, **characterized in that** it comprises 1 to 20% by weight of an alcohol.

13. Agent according to Claim 12, **characterized in that** the alcohol is 1,2,3-propanetriol.

14. Agent according to one of Claims 1 to 13, **characterized in that** it comprises at least one cationic polymer.

15. Agent according to one of Claims 1 to 14, **characterized in that** it comprises at least one amphoteric surface-active compound which is selected from carboxyl derivatives of imidazole, N-alkylamidobetaines, N-alkylsulphobetaines, N-alkylaminopropionates, alkyldimethylcarboxymethylammonium salts having 12 to 18 carbon atoms and fatty acid alkylamidobetaines.

16. Agent according to one of Claims 1 to 15, **characterized in that** it has a viscosity of from 100 to 30 000 mPa · s, measured at 25° Celsius using a Haake rotary viscometer model VT 550 at a shear rate of 12.9 per second.

17. Agent according to one of Claims 1 to 16, **characterized in that** it is in the form of a two-component preparation and is prepared directly prior to use by mixing the pure polymeric thickener or a composition comprising the polymeric thickener (component 1) with an aqueous hydrogen peroxide solution (component 2).

## Revendications

1. Agent transparent sous forme de gel pour le traitement oxydatif, comprenant
a) au moins un oxydant,
b) au moins un agent stabilisateur de peroxyde,
c) de 0,5 à 3,0 % en poids d'au moins un agent épaississant polymère choisi parmi les groupes
(i) les copolymères de l'acide acrylique, choisis parmi le groupe constitué d'un copolymère d'acrylate d'alkyle en C₁₀₋₃₀ et d'acide acrylique, d'acide méthacrylique ou de leurs esters simples, réticulé avec un éther allylique du sucrose ou un éther allylique du pentaérythritol; d'un copolymère d'acrylamide et d'acide acrylique, d'acide méthacrylique ou de leurs esters simples ; d'un copolymère d'acryloyldiméthyltaurate d'ammonium et de méthacrylate de béhéneth-25 ; d'un copolymère d'acryloyldiméthyltaurate d'ammonium et de vinylpyrrolidone ; et d'un homopolymère d'acide acrylique et d'acide méthacrylique, réticulé avec un éther allylique du sucrose, du pentaérythritol ou du propylène ;
(ii) les polymères renfermant des groupes propylène-oxy ;
(iii) les hydroxyalkylphosphates d'amidon et
(iv) les silicates de métal alcalin et de magnésium,
ainsi que
d) de l'eau ou un solvant aqueux.

2. Agent selon la revendication 1, **caractérisé en ce que** l'oxydant est choisi parmi le peroxyde d'hydrogène, des bromates de métaux alcalins, des bromates de métaux alcalino-terreux, le bromate d'ammonium, des persulfates de métaux alcalins, des persulfates de métaux alcalino-terreux, le persulfate d'ammonium, des perborates de métaux alcalins, des perborates de métaux alcalino-terreux, le perborate d'ammonium, des percarbonates de métaux alcalins, des percarbonates de métaux alcalino-terreux, le peroxyde de calcium et l'iodate de sodium.

3. Agent selon l'une quelconque des revendications 1 ou 2, **caractérisé en ce qu'**il comprend un bromate en tant qu'oxydant et présente un pH compris entre 7 et 8,5.

4. Agent selon l'une quelconque des revendications 1 à 3, **caractérisé en ce qu'**il présente un pH de 2 à 6.

5. Agent selon l'une quelconque des revendications 1 à 4, **caractérisé en ce que** l'oxydant est présent en une quantité de 0,1 à 25 pour cent en poids.

6. Agent selon l'une quelconque des revendications 1 à 5, **caractérisé en ce que** l'agent stabilisateur est choisi parmi des hydrogénophosphates de di-métaux alcalins, le p-acétamidophénol, des sels d'oxyquinoléine, l'acide salicylique et ses sels, l'acide 1-hydroxyéthane-1,1-diphosphonique, le 1-hydroxyéthane-1,1-diphosphonate de tétrasodium, l'iminodisuccinate de tétrasodium, l'acétate d'éthylènediamine-tétrasodium et le N-(4-éthoxyphényl)-acétamide.

7. Agent selon l'une quelconque des revendications 1 à 6, **caractérisé en ce que** l'agent stabilisateur est présent en une quantité de 0,01 à 2 pour cent en poids.

8. Agent selon l'une quelconque des revendications 1 à 7, **caractérisé en ce que** l'agent stabilisateur est une combinaison à 2 composants choisie parmi les suivantes:
l'ACIDE ETIDRONIQUE et l'ACIDE SALICYLIQUE,
l'ACIDE ETIDRONIQUE et le PHOSPHATE DE DISODIUM,
l'ETIDRONATE DE TETRASODIUM et l'ACIDE SALICYLIQUE,
l'ETIDRONATE DE TETRASODIUM et le PHOSPHATE DE DISODIUM.

9. Agent selon l'une quelconque des revendications 1 à 8, **caractérisé en ce que** l'agent stabilisateur est présent en une quantité de 0,1 à 0,3 % en poids et est choisi parmi l'ETIDRONATE DE SODIUM, l'ACIDE SALICYLIQUE et l'ACIDE ETIDRONIQUE.

10. Agent selon l'une quelconque des revendications 1 à 9, **caractérisé en ce que** l'agent épaississant polymère est choisi parmi le tableau suivant:
| N° | Nom INCI / CTFA | Désignation chimique |
|---|---|---|
| (9) | POLYMERE CROISE D'ACRYLATES/ACRYLATE D'ALKYLE EN C10-30 | Copolymère d'acrylate d'alkyle en C₁₀₋₃₀ et d'acide acrylique, d'acide méthacrylique ou de leurs esters simples, réticulé avec un éther allylique du sucrose ou un éther allylique du pentaérythritol |
| (10) | COPOLYMERE D'ACRYLATES / ACRYLAMIDE | Copolymère d'acrylamide et d'acide acrylique, d'acide méthacrylique ou de leurs esters simples (CAS n° 9003-06-9) |
| (11) | COPOLYMERE D'ACRYLOYLDIMETHYLTAURATE D'AMMONIUM / METHACRYLATE DE BEHENETH-25 | Copolymère d'acryloyldiméthyltaurate d'ammonium et de méthacrylate de béhéneth-25 |
| (12) | COPOLYMERE D'ACRYLOYLDIMETHYLTAURATE D'AMMONIUM / VP | Copolymère d'acryloyldiméthyltaurate d'ammonium et de vinylpyrrolidone |
| (13) | CARBOMERE | Homopolymère d'acide acrylique, d'acide méthacrylique, réticulé avec un éther allylique du sucrose, du pentaérythritol ou du propylène |
| (14) | HYDROXYPROPYLPHOSPHATE D'AMIDON | Ether hydroxypropylique de phosphate de diamidon |
| (15) | POLYQUATERNIUM-44 | Copolymère de méthylsulfate de 3- |
| | | méthyl-1-vinyl-1H-imidazolium et de 1-vinyl-2-pyrrolidone |
| (16) | POLYQUATERNIUM-37 | N,N,N-Triméthyl-2-[(méthyl-1-oxo-2-propényl)oxy]-chlorure, homopolymère |
| (17) | POLYQUATERNIUM-37, HUILE MINERALE, OLEATE DE SORBITANE, PEG-1 / PPG-6 TRIDECETH 6, ALCANE / CYCLOALCANE EN C10-12 | Mélange de N,N,N-triméthyl-2-[(méthyl-1-oxo-2-propényl)oxy]-chlorure, homopolymère; mono-oléate de sorbitane, éther tridécylique de polyéthylèneglycol / polypropylèneglycol, alcanes et cycloalcanes en C₁₀₋₁₂ (CAS 64742-48-9) |
| (18) | POLYQUATERNIUM-37, OLEATE DE SORBITANE, DICAPRYLATE / DICAPRATE DE PROPYLENEGLYCOL, PPG-1 TRIDECETH-6, ALCANE / CYCLOALCANE EN C10-12 | Mélange de N,N,N-triméthyl-2-[(méthyl-1-oxo-2-propényl)oxy]-chlorure, homopolymère; mono-oléate de sorbitane, ester dicaprylique de propylèneglycol (EINECS 271-516-3), éther tridécylique de polypropylèneglycol, alcanes et cycloalcanes en C₁₀₋₁₂ (CAS 64742-48-9) |
| (19) | SILICATE DE SODIUM ET DE MAGNESIUM | Silicate de sodium et de magnésium |

11. Agent selon l'une quelconque des revendications 1 à 10, **caractérisé en ce que** l'eau est présente en une quantité de 50 à 98 % en poids.

12. Agent selon l'une quelconque des revendications 1 à 11, **caractérisé en ce qu'**il comprend de 1 à 20 % en poids d'un alcool.

13. Agent selon la revendication 12, **caractérisé en ce que** l'alcool est le 1,2,3-propanetriol.

14. Agent selon l'une quelconque des revendications 1 à 13, **caractérisé en ce qu'**il comprend au moins un polymère cationique.

15. Agent selon l'une quelconque des revendications 1 à 14, **caractérisé en ce qu'**il comprend au moins un composé tensioactif amphotère qui est choisi parmi des dérivés carboxyliques de l'imidazole, des N-alkylamidobétaïnes, des N-alkylsulfobétaïnes, des N-alkylaminopropionates, des sels d'alkyldiméthylcarboxyméthylammonium renfermant de 12 à 18 atomes de carbone et des alkylamidobétaïnes d'acides gras.

16. Agent selon l'une quelconque des revendications 1 à 15, **caractérisé en ce qu'**il présente une viscosité de 100 à 30 000 mPa ·s, mesurée à 25° Celsius avec un viscosimètre rotatif Haake du type VT 550, à une vitesse de cisaillement de 12,9 par seconde.

17. Agent selon l'une quelconque des revendications 1 à 16, **caractérisé en ce qu'**il se présente sous la forme d'une préparation à 2 composants et est préparé directement avant l'application en mélangeant l'agent épaississant polymère pur ou une composition contenant l'agent épaississant polymère (composant 1) avec une solution aqueuse de peroxyde d'hydrogène (composant 2).
